Europäisches Patentamt

European Patent Office

Office européen des brevets

(19)

(11) Numéro de publication: **0 297 191 B1**

(12) **FASCICULE DE BREVET EUROPEEN**

(45) Date de publication de fascicule du brevet:
04.09.91

(51) Int. Cl.⁵: **A61K 31/40**

(21) Numéro de dépôt: 87401496.2

(22) Date de dépôt: 30.06.87

(54) **Application du N-[(1-éthyl 2-pyrrolidinyl) méthyl] 2-méthoxy 5-sulfamoyl benzamide au traitement de la stérilité.**

(43) Date de publication de la demande:
04.01.89 Bulletin 89/01

(45) Mention de la délivrance du brevet:
04.09.91 Bulletin 91/36

(84) Etats contractants désignés:
AT BE CH DE ES FR GB GR IT LI LU NL SE

(56) Documents cités:

SEM. HOP., vol. 55, no. 5/6, 1979, pages 296-297, Paris, FR; C. DEYDIER-FEISTHAUER: "Un cas de stérilité traité par le sulpiride"

FERTILITY AND STERILITY, vol. 41, no, 5, mai 1984, pages 714-718, The American Fertility Society, US; R. ZIMMERMANN et al.: "Preliminary report about a modified gonadotropin (human menopausal gonadotropin/human chorionic gonadotropin) treatment in infertile patients with premature luteinization"

GYNAEKOLOGE, vol. 18, no. 2, 1985, pages 54-62, Springer-Verlag; W. DISTLER et al.: "Diagnostik und Behandlung endokriner und tubarbedingter Sterilitätsursachen bei der Frau"

(73) Titulaire: **SOCIETE D'ETUDES SCIENTIFIQUES ET INDUSTRIELLES DE L'ILE-DE-FRANCE**
46, Boulevard de Latour-Maubourg
F-75340 Paris Cédex 07(FR)

(72) Inventeur: **Howie, Pr. Peter William**
**8 Travebank Gardens**
**Monifieth Dundee DD5 4ET(GB)**
Inventeur: **McNeilly, M. Alan**
**11 Craigend Road Stow**
**Galashiels Selkirkshire TDE 2RJ(GB)**

## Description

La présente invention concerne l'utilisation du sulpiride à des doses comprises entre 1 et 10 mg par jour, et en particulier à la dose de 3 mg par jour, pour le traitement de stérilités liées à des troubles du développement folliculaire, avec ou sans aménorrhée.

Le sulpiride, ou N-[(1-éthyl 2-pyrrolidinyl) méthyl] 2-méthoxy 5-sulfamoyl benzamide, est connu comme neuroleptique désinhibiteur dont la principale indication thérapeutique est le traitement des psychoses aiguës ou chroniques.

Les caractéristiques et les propriétés de ce composé sont décrites en particulier dans les brevets français 1472025 et 5916 M. Son utilisation, en tant que psychotrope, dans le traitement d'une stérilité masculine liée à des troubles psychiques, a été décrite dans Sem. Hôp., vol. 55, n° 5/6, (1979), 296-297.

L'utilisation du sulpiride, à des doses quotidiennes de 100 à 200mg, en association avec un traitement par les gonadotrophines hMG/hCG, dans le traitement de certains types de stérilités, a été décrite dans Fertility and Sterility, vol. 41, n° 5 (1984), 714-718.

D'autre part, différentes publications ont fait état d'un effet contraceptif du sulpiride. En particulier, l'effet contraceptif du sulpiride à des doses quotidiennes de 50 à 800mg (100mg dans la majorité des cas étudiés) a été décrit dans Revue française de Gynécologie, vol.71, n° 1, (1976), 53-61, cet effet étant attribué à l'action hyperprolactinémiante de ce composé.

L'effet contraceptif d'une association de progestatif et de sulpiride à faible dose, s'exerçant dans des conditions de fiabilité et de tolérance satisfaisantes, a également été décrit récemment.

On a cherché à savoir si l'effet contraceptif se maintenait avec le sulpiride utilisé seul, à très faibles doses.

Dans ce but, on a administré du sulpiride à des femmes volontaires, tous les jours, au coucher. Le sulpiride a été administré pendant toute la durée d'un cycle, un placebo ayant été administré pendant toute la durée du cycle précédent.

La présence ou l'absence d'ovulation était appréciée par dosage, dans un recueil des premières urines matinales, de LH, d'oestrone-3 glucuronate et de pregnanediol-3-alpha-glucuronate.

Le développement du follicule et la formation éventuelle d'un kyste folliculaire étaient appréciés par scannographie ultrasonore abdominale. Le taux sérique de prolactine était évalué au quatorzième jour du cycle.

La plupart des volontaires étaient des femmes normalement réglées, ovulant régulièrement. Mais pour pouvoir établir une comparaison des effets mesurés, les inventeurs ont élargi leur recrutement. Ils ont donc inclu dans le groupe étudié, trois femmes stériles, dont l'âge était compris entre 21 et 29 ans, présentant une aménorrhée depuis 36 mois en moyenne (23 à 51 mois).

Cette aménorrhée n'avait pas de cause déterminée : pas d'hyperprolactinémie ni d'anomalie de la selle turcique, pas de prise antérieure de médicaments antidopaminergiques ou oestro-progestatifs, poids corporels dans les limites normales.

Les taux de gonadotrophines étaient normaux et il n'y avait pas d'anomalie des fonctions thyroïdiennes ou surrénaliennes.

Quatre autres femmes étaient traitées pour une stérilité dont l'origine était attribuée à une phase lutéale courte. La durée de leurs cycles était courte et l'excrétion de prégnanediol était anormalement basse.

Contre toute attente, aux doses de sulpiride comprises entre 2 et 4 mg/jour, et en particulier à la dose de 3 mg/jour, il n'y a pas eu d'inhibition de l'ovulation chez les femmes réglées. Au contraire, par comparaison avec le cycle sous placébo, on a trouvé, durant le cycle sous sulpiride, une excrétion d'oestrone et de prégnanediol plus élevée alors que le taux de prolactine avait augmenté considérablement.

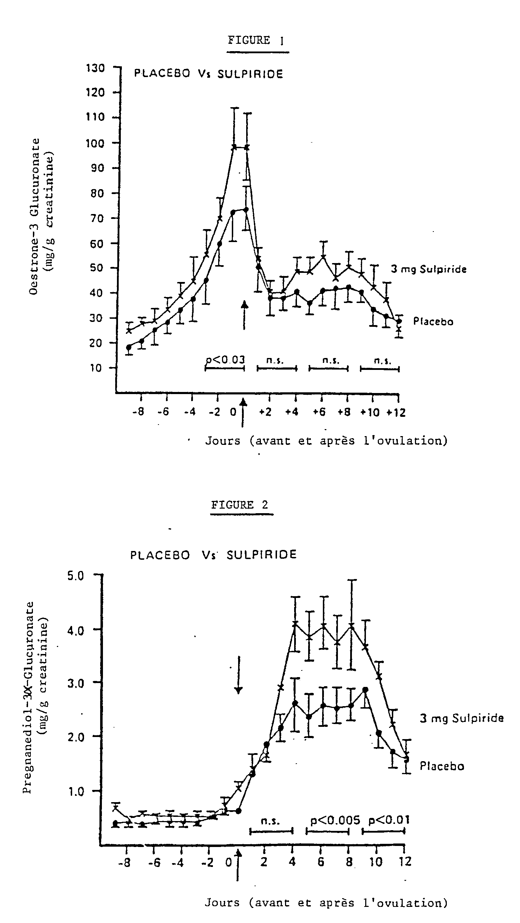

Les taux de ces hormones sont représentés sur les figures 1 et 2 ainsi que dans le tableau ci dessous :

2

Taux sériques de prolactine (m UI/1)

|  | PLACEBO | 3 mg SULPIRIDE |
|---|---|---|
| SUJET : 1 | 324 | 723 |
| 3 | 194 | 246 |
| 6 | 127 | 296 |
| 7 | 249 | 620 |
| 8 | 102 | 260 |
| 9 | 276 | 365 |
| 10 | 196 | 1441 |

On a surtout trouvé que deux des trois femmes aménorrhéiques avaient ovulé, ce qui était attesté par une excrétion biphasique d'oestrone et un pic de LH en milieu de cycle.

Chez les quatre femmes qui présentaient une phase lutéale courte, l'excrétion de prégnanediol ainsi que la durée du cycle se sont normalisées.

La poursuite de l'administration de sulpiride, à la même dose quotidienne, chez l'une des femmes aménorrhéiques qui souhaitait concevoir, à abouti à une grossesse dans les six mois suivants.

Trois des quatre femmes ayant une stérilité par insuffisance lutéale, ont également continué la prise de sulpiride. Chez deux d'entre d'elles, une grossesse a été obtenue dans le même délai de six mois. Le traitement a été parfaitement toléré.

Des essais réalisés avec des doses inférieures à 1 mg/jour et supérieures à 10 mg/jour n'ont pas produit des effets aussi satisfaisants sur le développement folliculaire, jugé d'après les taux d'excrétion des stéroïdes ovariens.

Selon la présente invention, on a préparé des comprimés contenant 2 à 4 mg (de préférence 3 mg) de sulpiride par dose unitaire et des excipients usuels tels que : amidon, dérivés de cellulose, talc.

On a également préparé des gélules contenant la même dose unitaire de sulpiride ou de l'un de ses sels pharmacologiquement acceptables. Chacune de ces formes convient pour obtenir les résultats recherchés.

## Revendications

1. Utilisation du N-[(1-éthyl 2-pyrrolidinyl) méthyl] 2-méthoxy 5-sulfamoyl benzamide ou de l'un de ses sels pharmacologiquement acceptables, à la dose unitaire de 1 à 10 mg, comme seul principe actif pour la préparation d'un médicament pour le traitement de la stérilité chez la femme.

2. Selon la revendication 1, utilisation du N-[(1-éthyl 2-pyrrolidinyl) méthyl] 2-méthoxy 5-sulfamoyl benzamide pour la préparation de comprimés et de gélules contenant 1 à 10 mg de principe actif par dose unitaire.

3. Selon la revendication 2, utilisation du N-[(1-éthyl 2-pyrrolidinyl) méthyl] 2-méthoxy 5-sulfamoyl benzamide pour la préparation de comprimés et de gélules contenant 2 à 4 mg de principe actif par dose unitaire.

4. Selon la revendication 3, utilisation du N-[(1-éthyl 2-pyrrolidinyl) méthyl] 2-méthoxy 5-sulfamoyl benzamide pour la préparation de comprimés et de gélules contenant 3 mg de principe actif par dose unitaire.

## Claims

1. Use of N-[(1-ethyl 2-pyrrolidinyl) methyl] 2-methoxy 5-sulfamoyl benzamide or one of its pharmacologically acceptable salts, in a unit dose of 1 to 10mg, as the sole active principle in the preparation of a medicament for treating sterility in women.

2. According to claim 1, the use of N-[(1-ethyl 2-pyrrolidinyl) methyl] 2-methoxy 5-sulfamoyl benzamide for the preparation of tablets and capsules containing 1 to 10mg of active principle per unit dose.

3. According to claim 2, the use of N-[(1-ethyl 2-pyrrolidinyl) methyl] 2-methoxy 5-sulfamoyl benzamide for the preparation of tablets and capsules containing 2 to 4mg of active principle per unit dose.

4. According to claim 3, the use of N-[(1-ethyl 2-pyrrolidinyl) methyl] 2-methoxy 5-sulfamoyl benzamide for the preparation of tablets and capsules containing 3mg of active principle per unit dose.

**Patentansprüche**

1. Verwendung von N-[(1-Ethyl-2-pyrrolidinyl)methyl]-2-methoxy-5-sulfamoyl-benzamid oder eines seiner pharmakologisch akzeptablen Salze in einer Einzeldosierung von 1 bis 10 mg als alleiniger Wirkstoff für die Herstellung eines Medikaments zur Behandlung der Sterilität bei der Frau.

2. Verwendung von N-[(1-Ethyl-2-pyrrolidinyl)methyl]-2-methoxy-5-sulfamoyl-benzamid nach Anspruch 1 für die Herstellung von Tabletten und Kapseln, die 1 bis 10 mg Wirkstoff pro Einzeldosierung enthalten.

3. Verwendung von N-[(1-Ethyl-2-pyrrolidinyl)methyl]-2-methoxy-5-sulfamoyl-benzamid nach Anspruch 2 für die Herstellung von Tabletten und Kapseln, die 2 bis 4 mg Wirkstoff pro Einzeldosierung enthalten.

4. Verwendung von N-[(1-Ethyl-2-pyrrolidinyl)methyl]-2-methoxy-5-sulfamoyl-benzamid nach Anspruch 3 für die Herstellung von Tabletten und Kapseln, die 3 mg Wirkstoff pro Einzeldosierung enthalten.

FIGURE 1

PLACEBO Vs SULPIRIDE

Jours (avant et après l'ovulation)

FIGURE 2

PLACEBO Vs SULPIRIDE

Jours (avant et après l'ovulation)